# EUROPÄISCHE PATENTSCHRIFT

(11) **EP 0 479 222 B1**
(45) Veröffentlichungstag und Bekanntmachung des Hinweises auf die Patenterteilung: **14.09.1994**
(21) Anmeldenummer: 91116738.5
(22) Anmeldetag: 01.10.1991
(51) Int. Cl.: C07D 307/58, A23L 1/226

(54) **Neuer Riech- und/oder Geschmackstoff**
Aromatic substances and/or flavours
Composés aromatiques et/ou d'assaisonnement

(30) Priorität: 04.10.1990 CH 3193/90
(43) Veröffentlichungstag der Anmeldung: 08.04.1992
(73) Patentinhaber: GIVAUDAN-ROURE (INTERNATIONAL) S.A., CH-1214 Vernier, Genève (CH)
(72) Erfinder: Müller, Peter M., CH-4106 Therwil (CH); Wild, Hans-Jakob, CH-8633 Wolfhausen (CH)
(74) Vertreter: Urech, Peter, Dr.

(56) Entgegenhaltungen:
- EP-A- 0 152 027
- US-A- 4 968 817
- TETRAHEDRON LETTERS Bd. 30, Nr. 45, 1989, GREATBRITAIN Seiten 6109 - 6112; F.W.J,DEMNITZ: 'The Mukaiyama reaction of ketenebis(trimethylsilyl)acetals with alpha-halo acetals a convenient butenolidesynthesis'
- TETRAHEDRON LETTERS Nr. 50, 1977, GREAT BRITAINSeiten 4443 - 4446; DAVID R.GEDGE ET AL.: 'Prenylations of but-2-enolides:Syntheses of Rosefuran and related natural Furans'

## Beschreibung

Die Erfindung betrifft einen neuen Riech- und/oder Geschmackstoff. Es handelt sich dabei um die Verbindung der Formel 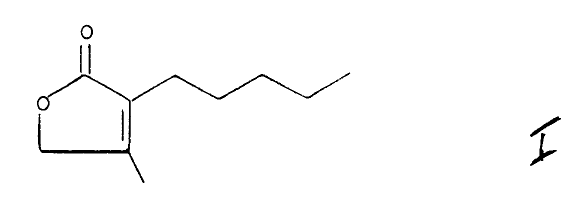
also um das 4-Methyl-3-pentyl-2(5H)-furanon.

Die Verbindung I weist besondere organoleptische Eigenschaften auf, auf Grund derer sie sich vorzüglich als Riech- und/oder Geschmackstoff eignet.

Die Erfindung betrifft demgemäss auch die Verwendung der Verbindung I als Riech- und/oder Geschmackstoff und Riech- und/oder Geschmackstoffkompositionen mit einem Gehalt an I.

Die Erfindung betrifft auch ein Verfahren zu deren Herstellung. Dieses Verfahren ist dadurch gekennzeichnet, dass man eine Verbindung der Formel 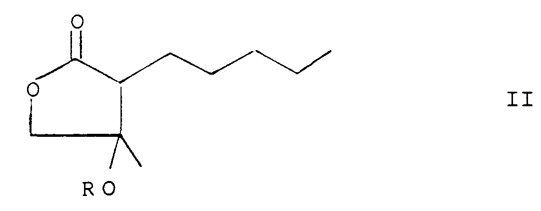
worin R Wasserstoff oder Acyl, z.B. C₁₋₅-Alkanoyl, z.B. Acetyl, bedeutet,
dehydratisiert.

Die Dehydratisierung der Verbindung II erfolgt zweckmässigerweise mittels Protonsäuren, z.B. mittels Mineralsäuren wie Salzsäure, Schwefelsäure, Phosphorsäure, Perchlorsäure, etc. oder, vorzugsweise, mittels organischen Sulfonsäuren, wie p-Toluolsulfonsäure, etc. Man arbeitet zweckmässigerweise in einem Lösungsmittel, wie einem Kohlenwasserstoff oder einem Alkohol oder Aether, und bei Zimmertemperatur, oder, vorzugsweise, bei erhöhter Temperatur.

Die Verbindungen der Formel II sind neu und bilden einen weitern Gegenstand der vorliegenden Anmeldung.

Die Herstellung einer solchen Verbindung II erfolgt beispielsweise aus
a) einem α-Halogenheptansäurealkylester und Hydroxyaceton, oder noch besser, einem Acylderivat, z.B. einem C₁₋₅-Alkanoyl oxyaceton, nach Reformatzky, oder
b) aus dem entsprechenden α-Halogencarbonsäureester, d.h. ∼ Heptansäureester von Hydroxyaceton mittels einer intramolekularen Reformatzkyreaktion.

Die erfindungsgemäss als Riech- und/oder Geschmackstoff verwendete Verbindung der Formel I zeichnet sich durch angenehme, ausgesprochen natürlich wirkende blumige und fruchtige Noten aus; sie sind kräftig und frisch. Die auftretenden Nebennoten sind: kokosnussartig, würzig, tabakartig. Zudem zeichnen sie sich durch ein hohes Diffusionsvermögen, verbunden mit einer ungewöhnlichen Haftfestigkeit aus. Bereits in geringen Konzentrationen eingesetzt, verstärken und veredeln sie das Geruchsbild von Riechstoffkompositionen, insbesondere von blumigen und fruchtigen Kompositionen oder Basen. Die Verbindung I verbindet sich mit zahlreichen bekannten Riechstoffingredienzien natürlichen oder synthetischen Ursprungs, wobei die Palette der natürlichen Rohstoffe sowohl leicht- als auch mittel- und Schwerflüchtige Komponenten, und diejenige der Synthetika Vertreter aus praktisch allen Stoffklassen umfassen kann, wie dies aus der folgenden Zusammenstellung ersichtlich ist:
- Naturprodukte: Basilikumöl, Baummoos Absolue, Beifussöl, Bergamotteöl, Cassisknospen Absolue, Castoreum, Cedernholzöl, Ciste Labdanum, Civette, Corianderöl, Eichenmoos, Elemiöl, Fichtennadelöl, Galbanum, Geraniumöl, Gewürznelkenöl, Jasmin Absolue und sein synthetischer Ersatz, Jonquille Absolue, Kamillenöl, Labdanum, Lavendelöl, Mandarinenöl, Mastix Absolue, Mentha citrata-öl, Myrrhenöl, Palmarosaöl, Patchouliöl, Petitgrainöl Paraguay, Sandelholzöl, Thymianöl, Vassouraöl, Moschus Infusion, Styrax, Birkenteer, Vetiveröl, Weihrauch, Ylang-Ylang-Oel, Zitronenöl, Zibetöl, etc.
- Alkohole: Citronellöl, Dimetol® (2,6-Dimethyl-heptan-2-ol), Geraniol, cis-3-Hexanol, Linalool, Nonadyl® (6,8-Dimethyl-nonan-2-ol), Phenyläthylalkohol, Rhodinol, Sandela® (3-Isocamiphyl-5-cyclohexanol), Sandalore® (3-Methyl-5-(2',2',3'-trimethyl-cyclopenta-3'-en-1'-yl)-pentan -2-ol), Terpineol, etc.
- Aldehyde: α-Amylzimtaldehyd, Cyclamenaldehyd, Decanal, Dodecanal, Heliotropin, α-Hexylzimtaldehyd, Hydroxycitronellal, Lyral, Adoxal® (2,6,10-Trimethyl-9-en-1-al), Undecanal, ω-Undecylenaldehyd, Vanillin, etc.
- Ketone: Isoraldeine® (Isomethyl-α-jonon), α-Jonon, β-Jonon, 3-Phenylisocaranon, Vertofix® (=acetyliertes Cedernholzöl), p-Methylacetophenon, etc.
- Ester: Acetessigsäureäthylester, Amylsalicylat, Benzylacetat, Cedrylacetat, Cinnamylformiat, Citronellylacetat, Geranylacetat, cis-3-Hexenylacetat, cis-3-Hexenylbenzoat, Linalylacetat, Linalylanthranilat, Methyldihydrojasmonat, Methambrat® (1-Acetoxy-1-methyl-2-sec.-butylcylohexan), Myraldylacetat® (4-(4-Methyl-3-pentenyl)-cyclohex-3-en-1-yl-carbinylacetat), Phenoxyäthylisobutyrat, Phenyläthyltiglat, Styrallylacetat, Terpenylacetat, 2,3,6,6-Tetramethylcyclohexy-2-en-carbonsäure-äthylester, 3,6,6-Trimethyl-2-äthyl-cyclohex-2-en-carbonsäure-äthylester, Vetivenylacetat, ortho-tert.Butylcyclohexyl-acetat, etc.
- Verschiedene: Ambrettemoschus, Cumarin, Epoxycedren, Eugenol, Fixolide® (1,1,2,4,4,7-Hexamethyl-6-acetyl -1,2,3,4-tetrahydronaphthalin), Galaxolid® (1,3,4,6,7,8-Hexahydro-4,6,6,7,8,8-hexamethyl-cyclopenta -γ-2-benzopyran), Heliotropin, Indol, Indolen, Isoeugenol, Isobutylchinolin, Jasmonyl (1,3-Diacetoxy-nonan), Ketonmoschus, Limonen, p-Menthan-8-thiol-3-on, Madrox® (1-Methylcyclododecyl-methyläther), Methyleugenol, Musk 174® (12-Oxahexadecanolid), γ-Nonalacton, α-Undecalacton, etc.

Die Verbindung der Formel I lässt sich in weiten Grenzen einsetzen, die beispielsweise von 0,1 (Detergentien) - 30 Gew.-% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen 0,1 und 25%. Die mit I hergestellten Kompositionen lassen sich für alle Arten von parfümierten Verbrauchsgütern einsetzen (Eaux de Cologne, Eaux de Toilette, Extraits, Lotionen, Cremes, Shampoos, Seifen, Salben, Puder, Zahnpasten, Mundwässer, Desodorantien, Detergentien, Tabak, etc.).

Die Verbindung I kann demgemäss bei der Herstellung von Kompositionen und - wie obige Zusammenstellung zeigt - unter Verwendung einer breiten Palette bekannter Riechstoffe bzw. Riechstoffgemische verwendet werden. Bei der Herstellung solcher Kompositionen können die oben angeführten bekannten Riechstoffe bzw. Riechstoffgemische nach (dem Parfümeur bekannter) Art und Weise verwendet werden, wie z.B. aus W.A. Poucher, Perfumes, Cosmetics, Soaps 2, 7. Auflage, Chapman und Hall, London 1974 hervorgehend.

Die neue Verbindung der Formel I ist ebenfalls vorzüglich geeignet zur Verwendung in Aromen verschiedenster Art, insbesondere aber auch zur Aromatisierung von Tabak.

Als Geschmackstoff kann die Verbindung I beispielsweise zur Erzeugung bzw. Verbesserung, Verstärkung, Steigerung oder Modifizierung von (tropischen) Fruchtaromen, z.B. Aprikosen-, Pfirsich-, Mango-, Guava-, Passionsfrucht-, Ananas-, Bananenaromen verwendet werden. Aber auch Nuss-, Kokosnuss-, Caramel-, Feldahorn, Tee-, Tabak-aromen kommen in Frage. Als Anwendungsgebiet dieser Aromen kommen beispielsweise Nahrungsmittel (Joghurt, Süsswaren etc.), Genussmittel (Tee, Tabak, etc.) und Getränke (Limonade etc.) in Frage.

Die ausgeprägten geschmacklichen Qualitäten der Verbindung I [süss, coumarinartig, laktonisch, nach Caramel; leicht fruchtig, frisch] ermöglichen die Verwendung als Aromastoff in geringen Konzentrationen. Eine geeignete Dosierung umfasst beispielsweise den Bereich von 0,1 ppm - 100 ppm, vorzugsweise den Bereich von 0,5 ppm - 50 ppm im Fertigprodukt, d.h. dem aromatisierten Nahrungsmittel, Genussmittel oder Getränk.

Bei der Aromatisierung von beispielsweise Tabak kann die Dosierung jedoch auch höher liegen und einen grösseren Bereich umfassen, beispielsweise den Bereich von 1 bis 1000 ppm, vorzugsweise 30-100 ppm.

Die Verbindung kann auf übliche Weise mit den für Geschmackstoffkompositionen verwendeten Bestandteilen vermischt bzw. solchen Aromen zugesetzt werden. Unter den erfindungsgemäss verwendeten Aromen werden Geschmackstoffkompositionen verstanden, die sich auf an sich bekannte Art verdünnen bzw. in essbaren Materialien verteilen lassen. Sie enthalten beispielsweise etwa 0,1-10, insbesondere 0,5-3 Gew.%. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmässigerweise verwendeten bekannten Aromastoffe sind entweder in der obigen Zusammenstellung bereits enthalten oder können leicht der Literatur entnommen werden, wi z.B. J. Merory, Food Flavorings, Composition, Manufacture and Use, Second Edition, The Avi Publishing Company, Inc., Westport, Conn. 1968, oder G. Fenaroli, Fenroli's Handbook of Flavor Ingredients, Second Edition, Volume 2, CRC-Press, Inc. Cleveland, Ohio, 1975.

Für die Herstellung solcher üblicher Gebrauchsformen kommen beispielsweise folgende Trägermaterialien, Verdickungsmittel, Geschmackstoffverbesserer, Gewürze und Hilfsingredientien, etc. in Frage:
Gummi arabicum, Tragant, Salze oder Brauereihefe, Alginate, Carrageen oder ähnliche Absorbentien; Indole, Maltol, Dienale, Gewürzoleoresine, Raucharomen; Gewürznelken, Diacetyl, Natriumcitrat; Mononatriumglutamat, Dinatriuminosin-5'-monophosphat (IMP), Dinatriumguanosin-5-phosphat (GMP); oder spezielle Aromastoffe, Wasser, Aethanol, Propylenglykol, Glycerin, etc.

### Beispiel 1

a) 65,37 g (1 Mol) Zinkpulver und 3,27 g (50 mMol) Jod in 400 ml Toluol werden vorgelegt. Diese violette Suspension wird unter Rühren im Oelbad auf 80°C erwärmt und innert 80 Minuten mit einer Lösung von 118,55 g (500 mMol) Aethyl 2-bromheptanoat und 87,08 g (750 mMol) Acetoxyaceton in 100 ml Toluol versetzt. Die sofort einsetzende exotherme Reaktion kann durch Entfernen des Oelbades bei 80-90°C gehalten werden. Nach weiteren 90 Minuten bei 80°C wird das auf Raumtemperatur abgekühlte Reaktionsgemisch mit 400 ml Eiswasser und dann mit 120 ml 20%ige HCl versetzt und 30 Minuten verrührt. Dann wird im Scheidetrichter dreimal mit 500 ml Aether extrahiert. Die Aetherphasen werden mit 500 ml ges. NaHCO₃-Lösung und mit 500 ml ges. NaCl-Lösung gewaschen, über MgSO₄ getrocknet und am Rotationsverdampfer eingeengt. Das Rohprodukt destilliert bei 110-112°C/0,06 mbar und ergibt 49,64 g (43,5%) 4-Acetoxy-4-methyl-3-pentyl-tetrahydrofuran-2-on.
   - IR (fl.Film):: 1780s, 1740s, 1460m, 1370s, 1230s, 1025s cm⁻¹
   - NMR(CDCl₃) 200 MHz:: 0,9(t,3H), 1,3-1.8(m,8H), 1,54 und 1,70(2s,3H), 2,05 und 2,07(2s/3H), 2,33 und 2,78(2t/1H), 4,40 und 4,46(2q/2H) ppm.
   - MS (m/e):: 169, 158, 89(100%), 43.
b) 125,33 g (549 mMol) 4-Acetoxy-4-methyl-3-pentyl-tetrahydrofuran-2-on werden mit 2,61 g (13.7 mMol) p-Toluolsulfonsäure vorgelegt und unter Rühren während 4 Stunden bei 150°C gehalten. Die abgespaltene Essigsäure wird über eine Vigreuxkolonne abdestilliert. Die auf Raumtemperatur abgekühlte Reaktionslösung wird mit 500 ml Aether verdünnt und im Scheidetrichter mit 2x200 ml ges. NaCl-Lösung gewaschen. Trocknen der Aetherphase über MgSO₄ und Einengen am Rotationsverdampfer sowie Destillation bei 100-103°C/0,12 mbar liefert 71,34 g /77,3%) 4-Methyl-3-pentyl-2(5H)-furanon.
   - IR (fl.Film):: 1750s, 1680s, 1450m, 1090s, 1040s cm⁻¹
   - NMR(CDCl₃) 200 MHz:: 0,9(t,3H), 1,3(m,8H), 1,6(m,2H), 2,03(s/3H), 2,25(t/2H), 4,62(m/2H) ppm.
   - MS (m/e):: 168(M⁺) 153, 139, 126, 112(100%).

### Beispiel 2

a) 86,0 g (411,3 mMol) 2-Bromheptansäure werden in 600 ml Dimethylformamid vorgelegt und unter Eiskühlung tropft man innert 5 Minuten bei 20°C 49,95 g (493,6 mMol) Triäthylamin zu und rührt die hellgelbe Lösung 15 minuten bei Raumtemperatur. Nun werden 57,07 g (617 mMol) Chloraceton während 30 Minuten zugetropft und die gelbbraune Suspension 90 Minuten bei Raumtemperatur nachgerührt. Das Reaktionsgemisch wird auf 1 l 1N HCl und Eis gegossen und im Scheidetrichter mit 3x500 ml Aether extrahiert. Die organischen Phasen werden mit 2x200 ml Wasser gewaschen, über MgSO₄ getrocknet und am Rotationsverdampfer eingeengt. Destillation bei 86-88°C/0,05 mbar liefert 80,56 g (73,9%) Acetylmethyl-2-bromheptanoat.
   - IR (Film):: 1750s(Schulter), 1735s, 1370m, 1275m, 1145s cm⁻¹
   - NMR(CDCl₃) 200 MHz:: 0,9(t,3H), 1,3-1,6(m,6H), 1,9-2,2 (m,2H), 2,2(s,3H), 4,34(t,1H), 4,74(q,2H) ppm.
   - MS (m/e):: 194, 191, 185, 111, 83, 43(100%).
b) 39,61 g (606 mMol) Zink pulverisiert werden in 500 ml Tetrahydrofuran suspendiert. Dann werden 1,92 g (15 mMol) Jod addiert und bei 67°C innert 1 Stunde 80,34 g (303 mMol) Acetylmethyl-2-bromheptanoat in 100 ml Tetrahydrofuran zugetropft. Die graugrüne Suspension wird noch 1 Stunde bei Rückflusstemperatur nachgerührt. Das erkaltete Reaktionsgemisch wird auf 500 ml 1N HCl und Eis gegossen und 10 Minuten verrührt. Dann wird im Scheidetrichter mit 3x500 ml Essigester extrahiert. Die organischen Phasen werden mit MgSO₄ getrocknet, am Rotationsverdampfer eingeengt und am Hochvakuum getrocknet. Es resultieren 77,21 g 4-Hydroxy-4-methyl-3-pentyl-tetrahydrofuran-2-on.
   - IR (Film):: 3400m, 1750s, 1025m cm⁻¹
   - MS (m/e):: 187, 171, 169, 116, 101(100%), 99.
c) 77 g (413,4 mMol) 4-Hydroxy-4-methyl-3-pentyl-tetrahydrofuran-2-on werden mit 1,57 g (8,3 mMol) p-Toluolsulfonsäure versetzt und 2 Stunden auf 150°C erhitzt, wobei mit einem schwachen Stickstoffstrom das entstehende Wasser über eine Kolonne abdestilliert wird. Die erkaltete Reaktionslösung wird mit 300 ml Aether verdünnt und im Scheidetrichter 3x mit 100 ml Wasser gewaschen. Die Wasserphasen werden mit 100 ml Aether zurückgewaschen. Trocknen der organischen Phasen über MgSO₄, Einengen am Rotationsverdampfer und Flachdestillation über eine kurze Vigreuxkolonne bei 0,3 mbar liefert 44,63 g hellgelbe Flüssigkeit. Durch Destillation an Widmerkolonne bei 100°C/0,07 mbar werden 37,73 g (54,2%) 4-Methyl-3-pentyl-2(5H)-furanon gewonnen. IR-, NMR- und MS-Spektren sind identisch mit Beispiel 1b).

### Beispiel 3

In den Kompositionen a), b) und c) vermittelt die Verbindung I Volumen und Fülle, was sich insbesondere in den fruchtigen und blumigen Aspekten niederschlägt.
a) blumiger, kosmetischer Akkord:

| | Gewichtsteile | |
|---|---|---|
| I | | 20,00 |
| Benzylacetat | 100,00 | 100,00 |
| Geranylacetat | 100,00 | 100,00 |
| p-t-Butylcyclohexylacetat | 100,00 | 100,00 |
| Verdylacetat | 20,00 | 20,00 |
| Phenyläthylalkohol | 150,00 | 150,00 |
| Hexylzimtaldehyd | 100,00 | 100,00 |
| Bergamotteessenz | 200,00 | 200,00 |
| Cyclohexylallylpropionat | 1,00 | 1,00 |
| Dimetol | 20,00 | 20,00 |
| Dipropylenglykol | 30,00 | 10,00 |
| Gardenol (Methylphenylcarbinylacetat) | 2,00 | 2,00 |
| Isoeugenol | 2,00 | 2,00 |
| Linalool | 50,00 | 50,00 |
| Geraniumoxyd 10%/DIP | 5,00 | 5,00 |
| Petitgrain Ess. Paraguay | 20,00 | 20,00 |
| Benzylsalicylat | 100,00 | 100,00 |
| Total | 1000,00 | 1000,00 |

Verwendung: z.B. 2% in Basen für Seifen, in Hautkosmetika.
b) blumiger, frischer Akkord:

| | Gewichtsteile | |
|---|---|---|
| I | | 30,00 |
| Benzylacetat | 80,00 | 80,00 |
| Geranylacetat | 150,00 | 150,00 |
| Linalylacetat | 200,00 | 200,00 |
| p-t-Butylcyclohexylacetat | 100,00 | 100,00 |
| Agrumex (2-tert.Butylcyclohexylacetat) | 80,00 | 80,00 |
| Phenylpropylalkohol | 100,00 | 100,00 |
| Dipropylenglykol | 50,00 | 20,00 |
| Eugenol | 10,00 | 10,00 |
| Isoraldeine 70 P (Isomethyl-α-ionon) | 50,00 | 50,00 |
| Lemarome A (Neral-Citralgemisch) | 30,00 | 30,00 |
| Hexylsalicylat | 50,00 | 50,00 |
| Tetrahydrolinalool | 100,00 | 100,00 |
| Total | 1000,00 | 1000,00 |

Verwendung: z.B. 2% in einer Base für Douch-Gele.
c) blumiger, eleganter Akkord:

| | Gewichtsteile | |
|---|---|---|
| I | | 10,00 |
| Benzylacetat | 100,00 | 100,00 |
| Dimethylbenzylcarbinylacetat | 40,00 | 40,00 |
| Geranylacetat | 30,00 | 30,00 |
| Phenyläthylalkohol | 100,00 | 100,00 |
| Bergamotte -rekonstitution | 200,00 | 200,00 |
| Keton alpha (α-Methyl-ionon) | 50,00 | 50,00 |
| Cyclohexal | 50,00 | 50,00 |
| Dipropylenglykol | 10,00 | |
| Eugenol | 10,00 | 10,00 |
| Galaxolid 50 DEP | 100,00 | 100,00 |
| Gardenol | 10,00 | 10,00 |
| Geraniol extra | 60,00 | 60,00 |
| Hedione | 20,00 | 20,00 |
| Hydroxycitronellal | 60,00 | 60,00 |
| Methylisoeugenol | 10,00 | 10,00 |
| Benzylsalicylat | 150,00 | 150,00 |
| Total | 1000,00 | 1000,00 |

Verwendung: z.B. 15% in Alkohol 90°, für Eaux de toilette.
d) Haselnussaroma:

| | Gewichtsteile |
|---|---|
| 3,4-Dimethyl-cyclopenta-1,2-dion | 6,00 |
| Vanillin | 20,00 |
| 2-Acetyl-pyrazin | 2,00 |
| Propylenglykol | 953,00 |
| Diacetyl | 3,00 |
| Dimethyl-resorcinol | 3,00 |
| γ-Nonalacton (Aldehyd Cl) | 1,00 |
| Benzaldehyd | 2,00 |
| Furfural | 4,00 |
| Trimethyl-pyrazin | 1,00 |
| I | 5,00 |
| Total | 1000,00 |

Dosierung: z.B. 0,1% in milk drinks.
In obigem Haselnussaroma erbringen die 0,5 ppm Verbindung I kremigen Körper, Mundgefühl. Sie verstärken dadurch insbesondere den Nusscharakter des Aromas.
e) Chocolataroma:

| | Gewichtsteile |
|---|---|
| Vanillin | 80,00 |
| Phenylessigsäure | 3,00 |
| Aethyl-Vanillin | 50,00 |
| Cocoa Shell Extract PG (Kakaoschalenextrakt) | 850,80 |
| Benzaldehyd | 0,20 |
| iso-Buttersäure | 0,50 |
| Phenyläthylalkohol | 2,00 |
| Aethylphenylacetat | 1,50 |
| iso-Valerialdehyd | 2,00 |
| Aethyl-2-dimethyl-3,5(bzw. 6)-pyrazin | 2,00 |
| iso-Butyraldehyd | 1,00 |
| Trimethylpyrazin | 2,00 |
| I | 5,00 |
| Total | 1000,00 |

Dosierung: 0,1% in milk drinks, etc.
In obigem Chocolataroma erbringen die 0,5 ppm der Verbindung I Körper und Mundgefühl; sie verstärken den Charakter der Komposition.
f) Ananasaroma:

| | Gewichtsteile | |
|---|---|---|
| Aethylvalerat | 1 | 1 |
| γ-Decalacton | 1 | 1 |
| Aethylcapronat | 4 | 4 |
| Aethyl-2-methylbutyrat | 4 | 4 |
| 2-Methylbuttersäure | 5 | 5 |
| iso-Amylacetat | 9 | 9 |
| Aethylacetat | 10 | 10 |
| Methyl-2-methylbutyrat | 10 | 10 |
| Homofuronol 20% in PG | 15 | 15 |
| Aethylbutyrat | 16 | 16 |
| Allylcapronat | 25 | 25 |
| I | -- | 5 |
| Propylenglykol | 900 | 895 |
| Total | 1000 | 1000 |

Der Zusatz an Verbindung I bringt Körper, eine natürliche saftige und frische Note, mehr Fülle.
g) Passionsfruchtaroma:

| | Gewichtsteile | |
|---|---|---|
| Mandarinenöl | 0,500 | 0,500 |
| Phenäthylalkohol | 0,500 | 0,500 |
| Geraniol | 0,500 | 0,500 |
| cis-3-Hexenol | 0,500 | 0,500 |
| Citral | 0,500 | 0,500 |
| α-Jonon | 0,500 | 0,500 |
| γ-Undecalacton | 1 | 1 |
| Linalool | 1 | 1 |
| α-Terpineol | 1 | 1 |
| Linalylacetat | 1 | 1 |
| Styrallylacetat | 2 | 2 |
| Aethylbutyrat | 4 | 4 |
| Aethylisovalerat | 5 | 5 |
| Hexylcapronat | 14 | 14 |
| Homofuronol 20% in PG (2-Aethyl-4- | | |
| hydroxy-5-methyl-dihydrofuran- | | |
| 3(2H)-on) | 20 | 20 |
| Aethylcapronat | 38 | 38 |
| Hexylbutyrat | 30 | 30 |
| I | -- | 5 |
| Triäthylcitrat | 80 | 75 |
| Propylenglykol | 800 | 800 |
| Total | 1000 | 1000 |

Der Zusatz an I akzentuiert die frische und saftige Note, erbringt Fülle und Körper.
h) Aprikosenaroma:

| | Gewichtsteile | |
|---|---|---|
| Hexylacetat | 1 | 1 |
| β-Damascon 10% in PG | 1 | 1 |
| γ-Undecalacton | 2 | 2 |
| cis-3-Hexenol | 2 | 2 |
| Benzaldehyd | 3 | 3 |
| trans-2-Hexenol | 3 | 3 |
| Linalool | 5 | 5 |
| γ-Dodecalacton | 5 | 5 |
| Buttersäure | 10 | 10 |
| Aethylacetat | 10 | 10 |
| γ-Decalacton | 8 | 8 |
| Aethylpropionat | 10 | 10 |
| iso-Amylacetat | 10 | 10 |
| 2-Methylbuttersäure | 10 | 10 |
| Essigsäure | 20 | 20 |
| Homofuronol 20% in PG | 20 | 20 |
| I | -- | 5 |
| Propylenglykol | 880 | 875 |
| Total | 1000 | 1000 |

Der Zusatz an I erbringt Körper, Fülle, Abrundung, eine saftigere, fruchtigere und reife und frische Note.

## Patentansprüche (Patentansprüche für folgende(n) Vertragsstaat(en): CH, DE, FR, GB, IT, LI, NL)

1. 4-Methyl-3-pentyl-2(5H)-furanon.

2. 4-Hydroxy (bzw. Acyloxy)-4-methyl-3-pentyl-tetrahydrofuran-2-on.

3. Riech- und/oder Geschmackstoffkomposition, gekennzeichnet durch einen Gehalt an 4-Methyl-3-pentyl-2(5H)-furanon.

4. Verfahren zur Herstellung von 4-Methyl-3-pentyl-2(5H)-furanon, dadurch gekennzeichnet, dass man 4-Hydroxy (bzw. Acyloxy)-4-methyl-3-pentyl-tetrahydrofuran-2-on dehydratisiert.

5. Verwendung von 4-Methyl-3-pentyl-2(5H)-furanon als Riech- und/oder Geschmackstoff.

## Patentansprüche (Patentansprüche für folgende(n) Vertragsstaat(en): ES)

1. Riech- und/oder Geschmackstoffkomposition, gekennzeichnet durch einen Gehalt an 4-Methyl-3-pentyl-2(5H)-furanon.

2. Verfahren zur Herstellung von 4-Methyl-3-pentyl-2(5H)-furanon, dadurch gekennzeichnet, dass man 4-Hydroxy (bzw. Acyloxy)-4-methyl-3-pentyl-tetrahydrofuran-2-on dehydratisiert.

3. Verwendung von 4-Methyl-3-pentyl-2(5H)-furanon als Riech- und/oder Geschmackstoff.

## Claims (Claims for the following Contracting State(s): CH, DE, FR, GB, IT, LI, NL)

1. 4-Methyl-3-pentyl-2(5H)-furanone.

2. 4-Hydroxy (or acyloxy)-4-methyl-3-pentyltetrahydro-furan-2-one.

3. An odorant and/or flavorant composition, characterized by a content of 4-methyl-3-pentyl-2(5H)-furanone.

4. A process for the manufacture of 4-methyl-3-pentyl-2(5H)-furanone, characterized by dehydrating 4-hydroxy (or acyloxy)-4-methyl-3-pentyl-tetrahydrofuran-2-one.

5. The use of 4-methyl-3-pentyl-2(5H)-furanone as an odorant and/or flavorant.

## Claims (Claims for the following Contracting State(s): ES)

1. An odorant and/or flavorant composition, characterized by a content of 4-methyl-3-pentyl-2(5H)-furanone.

2. A process for the manufacture of 4-methyl-3-pentyl-2(5H)-furanone, characterized by dehydrating 4-hydroxy (or acyloxy)-4-methyl-3-pentyl-tetrahydrofuran-2-one.

3. The use of 4-methyl-3-pentyl-2(5H)-furanone as an odorant and/or flavorant.

## Revendications (Revendications pour l'(les) Etat(s) contractant(s) suivant(s): CH, DE, FR, GB, IT, LI, NL)

1. La 4-méthyl-3-pentyl-2(5H)-furannone.

2. Une 4-hydroxy-(ou acyloxy)-4-méthyl-3-pentyl-tétrahydrofuranne-2-one.

3. Composition odorante et/ou sapide, caractérisée en ce qu'elle contient de la 4-méthyl-3-pentyl-2(5H)-furanonne.

4. Procédé de préparation de la 4-méthyl-3-pentyl-2(5H)-furanonne, caractérisé en ce que l'on déshydrate une 4-hydroxy-(ou acyloxy)-4-méthyl-3-pentyl-tétrahydrofuranne-2-one.

5. Utilisation de la 4-méthyl-3-pentyl-2(5H)-furanonne en tant que matière odorante et/ou sapide.

## Revendications (Revendications pour l'(les) Etat(s) contractant(s) suivant(s): ES)

1. Composition odorante et/ou sapide, caractérisée en ce qu'elle contient de la 4-méthyl-3-pentyl-2(5H)-furanonne.

2. Procédé de préparation de la 4-méthyl-3-pentyl-2(5H)-furanonne, caractérisé en ce que en ce que l'on déshydrate une 4-hydroxy-(ou acyloxy)-4-méthyl-3-pentyl-tétrahydrofuranne-2-one.

3. Utilisation de la 4-méthyl-3-pentyl-2(5H)-furanonne en tant que matière odorante et/ou sapide.
